# EUROPEAN PATENT SPECIFICATION

(11) **EP 2 046 312 B1**
(45) Date of publication and mention of the grant of the patent: **19.02.2020**
(21) Application number: 07765188.3
(22) Date of filing: 13.07.2007
(51) Int. Cl.: A61K 31/122, A61K 31/231, A61K 31/232, A61K 35/60, A61P 9/00, A61P 19/00

(54) **PHARMACEUTICAL AND NUTRACEUTICAL PRODUCTS COMPRISING VITAMIN K2**
PHARMAZEUTISCHE UND NUTRAZEUTISCHE PRODUKTE MIT VITAMIN K2
PRODUITS PHARMACEUTIQUES ET NUTRITCEUTIQUES COMPRENANT DE LA VITAMINE K2

(30) Priority: 14.07.2006 NO 20063291; 14.07.2006 NO 20063292; 06.09.2006 NO 20063988
(43) Date of publication of application: 15.04.2009
(73) Proprietor: Kaydence Pharma AS, 0283 Oslo (NO)
(72) Inventor: VERMEER, Cees, 6200 MD Maastricht (NL); SCHURGERS, Leon, J., 6200 MD Maastricht (NL); KLAVENESS, Jo, 0203 Oslo (NO); VIK, Hogne, 0203 Oslo (NO); VIK, Anne, Bjornebye, 0203 Oslo (NO); WESTBYE, Stein, 0203 Oslo (NO)
(74) Representative: Weickmann & Weickmann PartmbB
(86) International application number: PCT/EP2007/006241
(87) International publication number: WO 2008/006607

(56) References cited:
- EP-A1- 1 153 548
- WO-A-02/01969
- WO-A-02/074308
- WO-A1-2004/019923
- WO-A1-2005/030190
- JP-A- 3 254 658
- JP-A- 2003 169 632
- JP-A- 2003 313 142
- JP-A- 2005 237 300
- SUZUKI T ET AL: "CASE-CONTROL STUDY OF RISK FACTORS FOR HIP FRACTURES IN THE J APANESE ELDERLY BY A MEDITERRANEAN OSTEOPOROSIS STUDY (MEDOS) QUESTIONNAIRE", BONE, PERGAMON PRESS., OXFORD, GB, vol. 21, no. 5, 1 November 1997 (1997-11-01), pages 461-467, XP003035051, ISSN: 8756-3282
- PLAZA S M ET AL: "Vitamin K2 in bone metabolism and osteoporosis", ALTERNATIVE MEDICINE REVIEW, THORNE RESEARCH INC., SANDPOINT, US, vol. 10, no. 1, 1 March 2005 (2005-03-01), pages 24-35, XP002621178, ISSN: 1089-5159

## Description

### Field of the invention

This invention relates to pharmaceutical and nutraceutical products comprising menaquinone-7 (MK-7) in combination with one or more of a polyunsaturated fatty acid, fish oil and krill oil in a tablet or capsule formulation wherein MK-7 is in the range of between 1-10 µg, and their use in the treatment or prophylaxis of disorders related to bone, cartilage and the cardiovascular system. In particular, the invention relates also to the use of such products in the treatment and/or prevention of osteoporosis, atherosclerosis and osteoarthritis.

### Background art

### Vitamin K₂

Vitamin K₂ is a group of compounds called menaquinones ("MK") which are all 2-methyl-3-all-trans-polyprenylated-1,4-naphthoquinones having the following structural formula:

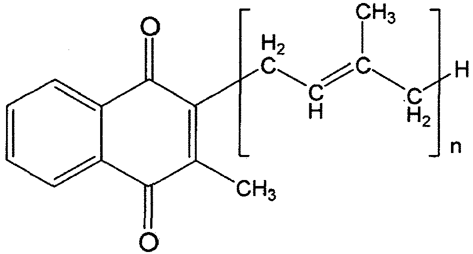

The chemical difference between the different MKs relates to the number of isoprene units in the side chain. The various MKs are normally referred to as MK-2, MK-3, MK-4, MK-6 and so on. The number refers to the number of isoprene units (n=2, n=3, n=4, n=6, ...).

The term vitamin K₂ refers to the naturally occurring mixture of the different MK substances. MK-2 through MK-13 are naturally present in animal and human tissue. Dietary sources of vitamin K₂ are typically fermented foods, notably cheese and curd cheese. Also certain flatfish and eel may contain some vitamin K₂. "Natto", which is produced from fermented soy beans, is a popular source of vitamin K₂ as a "health product" in Japan. The daily intake of vitamin K₂ can vary over a wide range and can typically be from a few µg to several milligrams; generally below 50 µg. Vitamin K₂ is claimed to have several beneficial effects for human health, mainly related to the cardiovascular system and bone metabolism.

### Osteoporosis

Osteoporosis is a medical condition characterized by decreased bone mass and changes in the micro architecture of the bone. It is estimated that more than 200 million woman have osteoporosis worldwide. Osteoporosis and osteoporotic fractures are increasing all over the world mainly as a result of the fast growing elderly population. Treatment of osteoporosis today includes typical hormone therapy, intake of calcium and vitamin D and drug treatment using bisphosphonates.

### Atherosclerosis

Atherosclerosis is a cardiovascular disease that can affect the arteries of several vital organs including brain, heart, kidneys as well as arms and legs. In most Western countries, atherosclerosis is the leading cause of death. The number of deaths from atherosclerosis is about twice the number of deaths from all cancer diseases together. Atherosclerosis is a slow, progressive disease which might start as early as in childhood. In atherosclerosis the arteries loose their elasticity and harden. There are several conditions associated with the development and progression of the disease including risk factors like obesity, diabetes, hypertension and smoking. Whereas strictly medically speaking atherosclerosis is generally defined as an inflammatory disease of the arterial tunica intima, arteriosclerosis is defined more broadly and also comprises other forms of vascular disease such as Mönckeberg's sclerosis of the media.

### Arthrosis and arthritis

Arthrosis, inflammatory arthritis, rheumatoid arthritis and osteoarthritis are diseases of the cartilage and they are characterized by articular degeneration. Pathologically, there is an alteration in the cartilage structure. These diseases affect the joints and the disease can often easily been diagnosed by X-ray. Several types of drugs are today used for treatment of these diseases. These include simple analgesics, NSAIDs, COX 2 inhibitors, corticosteroids and glucosamine.

### Effect of vitamin K₂ on osteoporosis, atheroslerosis, arthrosis and arthritis

During the last years several publications describe a positive effect of intake of vitamin K₂ on osteoporosis; see, for example, W. Sakamoto et al. in Osteoporosis International 16, 1604-1610 (2005); M. Kaneki in Clinical Calcium 15, 605-610 (2005); J. Iwamoto et al. in Current Pharmaceutical Design 10, 2557-2576 (2004); K. Nakayama in Horumon to Rinsho 52, 339-349 (2004); S. Shiomi et al. in American Journal of Gastroenterology 97, 978-981 (2002) and T. Hosoi in Bone (Osaka) 14, 95-97 (2000).

There are several publications related to the use of vitamin K₂ or Natto for treatment of atherosclerosis or similar conditions; see, for example, Y. Ozawa in Gifu Daigaku Igakubu 50, 20-26 (2002); L. J. Schurgers et al in Zeitschrift für Kardiologie 90 (Suppl. 3), iii57-iii63 (2001); Y. Seyama in Clinical Calcium 9, 873-878 (1999); H. Kawashima et al. in Jap. Journal of Pharmacology 75, 135-143 (1997) and J. M. Geleijnse et al. in the Journal of Nutrition 134, 3100-3105 (2004).

In a recent paper, T. Neoqi et al. (Arthritis & Rheumatism 54, 1255-1261 (2006)) reported that low levels of serum vitamin K are associated with an increased prevalence of osteoarthritis.

### Fish oil, krill oil and n-3 PUFA

Polyunsaturated fatty acids, or PUFAs, are long-chain fatty acids containing two or more double bonds. Interest in them arises from their potential in therapeutic applications, and food and nutritional applications. They occur throughout animal, plant, algae, fungi and bacteria and are found widely in many lipid compounds such as membranes, storage oils, glycolipids, phospholipids, sphingolipids and lipoproteins. They are produced commercially from selected seed plants, and some marine sources.

PUFAs are grouped into two series on the basis of the position of the terminal double bond being 3C or 6C from the terminal carbon atom of the fatty acid chain. Some examples are: The 3-series PUFA, also referred to as omega-3 or (n-3) PUFA, includes omega-3 fatty acid-rich dietary oils, such as fish oil, krill oil, eicosapentaenoic acid (EPA), docosahexaenoic asid (DHA), linolenic acid (LA), and alpha-linolenic acid (ALA). The 6-series PUFA includes gamma-linolenic acid (GLA) and arachidonic acid (AA).

Krill is a group of shrimp-like marine animals living in the Arctic and Antarctic regions. The size of krill is normally between 5 and 50 mm. There are several species of krill. The Arctic species include *T. inermis, T. rushii, T. longicauda* and *M*. *norvegia,* while the Antarctic species include *E*. *superba* and *E*. *crystallorphias.* Krill and krill related products have been suggested as components in feed for fish; see, for example, KR 2005031319, KR 2004087618, JP 2003070426, US 6153251, EP 729708, SU 1784152, JP 05030923, JP 0465454 and JP 61274653.

Furthermore, krill and krill-based products are used as fish baits (see, for example, CN 1820626), in combination with conjugated linoleic acid for treatment and prophylaxis of diseases (US 2006078625), as additives to inhibit oxidation of lipids (WO 2005075613), as fertilizer (CN 1502589), in foodstuff (JP 2004065152 and WO 2003/003857), for treatment of cardiovascular diseases, arthritis, skin cancer, diabetes and premenstrual syndrome (WO 2002/102394), as a source for enzyme for treatment of acne and other diseases (US 5958406, US 5945102, US 6030612, WO 96/24371 and WO 93/24142) and as a source for multifunctional enzymes (US 6030612). Krill enzymes have further been suggested for treatment of thrombosis (WO 95/33471) and in use for manufacture of compositions for dental use (WO 95/33470).

Krill or krill based-products are also suggested as fungicides (JP 07033619), as antidiabetic agents (JP 04042369), and proteins from krill have been suggested for manufacture of health foods (JP 01137952). Other patent documents related to krill and krill components are: dentifrices comprising krill fatty acids (JP 2568833), cosmetics containing proteases extracted from krill for skin cleansing (JP 63218610), antiinflammatory proteinase from krill (JP 61068419), thrombus dissolvent (EP 170115), enzyme composition as digestion promoter (WO 85/04809), chitin preparation (PL 114387), carotenoid preparations (PL 113328, crabmeat-like food (JP 57125677, JP 57079863 and JP 67050848), antihypertensives (JP 54119011) and krill-based food (JP 60046947, JP 61023987 and JP 55040218).

The total lipid content of krill varies from species to species. There are also variations of the total lipid content during the year. The lipid composition of krill also varies for each species during the year. The total lipid in krill is typically between 5 and 60%. The percentage of total triglycerols and wax esters of total lipid in krill can vary over a wide range; each from almost zero to 70%. In addition to triglycerols and wax esters, krill lipid, hereafter krill oil, comprises phospholipids, sterols, free fatty acids and fatty alcohols. Typical phospholipids include compounds like phosphatidylcholines and phosphatidyl-ethanolamines. The krill oil can be in the form of raw oil obtained from krill or in the form of purified or modified oil. Unsaturated compounds and polyunsaturated compounds form a large portion of the fatty compounds in krill oil. The main fatty acid constituents in krill oil are the following fatty acids: 14 : 0, 16 : 0, 16 : 1 (n-7), 18 : 0, 18 : 1, 18 : 1 (n-9), 18: 1 (n-7) , 18 : 2 15 (n-6), 18 : 3 (n-3), 18 :4 (n-3), 20 : 1 (n-9), 20 : 5 (n-3), 22 : 1 (n-11), 22 : 6 (n-3).

For references on composition of krill oil, see, for example: S. Falk-Perdersen et al. in Can. J. Fish Aquat. Sci. 57 (Suppl. 3) 178-191 (2000), F. Alonzo et al. in Marine Ecology: Progress Series 296, 65-79 (2005), N. Kusumoto et al. in J. Oleo Science 53, 45-51(2004) and references herein.

### Further prior art

JP 03254658A relates to a food product based on Natto blended with a substance, e.g. (semi-)dried material such as beans, grains, starch, fish meat, animal meat, marine algae or seasoning.

JP 2003169632A relates to products which increase the usefulness of health food by the synergism of the component substance contained in rice, embryo/germ oil and the component substance in egg yolk oil and phosphatidylserine.

JP 2003313142 relates to a liquid or powdery highly nutritional composition for use in nutrition to patients who are unable to take normal meal, especially aged persons and care-receivers. The nutritional composition comprises, as nutritional ingredients, lactoproteins, soybean proteins, lipids, dietary fibers, digosaccharides, catechins or polyphenols, vitamins, minerals and microelements.

WO 02/01969 A1 relates to a nutritional and pharmaceutical formulation comprising in combination vitamin K and a source of at least one essential fatty acid (EFA) as the essential ingredients, and foodstuff containing EFA(s) and an artificially elevated quantity of vitamin K are provided for the treatment or prevention of variety of diseases or conditions.

WO 02/074308 A1 relates to a composition useful in the prevention of osteoporosis, wherein said composition comprises (a) least one isoflavone and/or isoflavone glycoside, preferably genistein and/or genistin; (b) at least one polyunsaturated fatty acid; (c) optionally vitamin D, and/or one or more derivatives thereof and/or vitamin K and/or one or more derivatives thereof; and (d) optionally adjuvants and excipients in quantities as required, preferably within the range of 0.1 to 20 % by weight, based on the total weight of the composition.

JP 2005237300 relates to a foodstuff material and a method of manufacturing the same. The foodstuff is obtained by propagating Bacillus natto in a plant excerpt as soybean-related material to produce vitamin K.

WO 2004/019923 A1 relates to a pharmaceutical composition or nutritional formulation comprising vitamin K which can be used to combat age-related stiffening of the arteries, and the consequences thereof, namely pulmonary congestion, hypertension, left ventricular hypertrophy, congestive (right sided) heart failure, left sided or left ventricular failure, chronic cardiac failure, angina pectoris, myocardial infarction, Monckeberg's sclerosis and stroke.

Suzuki et al., 1997, Bone, 21(5): 461-467 relates to a case control study of hip fracture among the Japanese elderly which was carried out in order to assess the risk factors for fractures. The authors conclude that some traditional Japanese lifestyle characteristics may prevent hip fractures among the Japanese elderly.

EP 1 153 548 A1 relates to a food product comprising menaquinone at a level of 50 to 5000 µg/100g of product.

WO 2005/030190 A1 relates to pharmaceutical compositions and nutritional supplements comprising an effective daily dose of menaquinone-7.

Plaza et al., 2005, Alternative Medicine Review 10(1): 24-35 relates to *in vitro, in vivo* and human data on the positive effect of vitamin K₂ on osteoporosis.

Although there are several pharmaceutical products on the market for treatment of osteoporosis and cardiovascular disease, there still exists an urgent need for alternative approaches.

### Summary of the invention

The scope of the invention is determined by the appended claims.

It has now been surprisingly found that vitamin K₂ and polyunsaturated fatty acids (PUFAs) can be formulated together in a stable formulation with long shelf life.

Thus, in a first aspect, the present invention provides a pharmaceutical or nutraceutical product for oral administration comprising MK-7
in combination with at least one of a PUFA, either purified or in the form of fish oil or krill oil wherein said product is a tablet formulation or a capsule formulation and wherein MK-7 is in the range of between 1-10 µg.

It was also found that vitamin K₂, and in particular MK-7, does not counteract the anticoagulant effect of marine oils or n-3 PUFA.

It was further found that the higher menaquinones, in particular MK-7 and higher, counteract potential artery calcification-inducing effects of marine oils.

It was further found that higher menaquinones, in particular MK-7 and higher, counteract potentially negative aspects of marine oils on bone health.

It was further found that higher menaquinones, in particular MK-7 and higher, counteract potentially negative aspects of marine oils on cartilage health.

It was further found, in cell culture studies, that MK-7 is taken up better than other K vitamins. MK-8 and MK-9 are taken up somewhat less, but at a comparable level, whereas MK-4 and vitamin K₁ are taken up to a much lower extent.

It was further found, in cell culture studies, that the long-chain menaquinones, in particular MK-8 and MK-9, are less likely to interfere with oral anticoagulant treatment than other forms of vitamin K.

Thus, in a second aspect, the present invention provides a pharmaceutical or nutraceutical product comprising MK-7 in combination of at least one of a PUFA, either purified or in the form of fish oil or krill oil, for use in a method for preventing or treating at least one of cardiovascular-, bone- and cartilage-related diseases or disorders in humans and animals , wherein the dosage of MK-7 is in the range of between 1-10 µg per day.

Further described is the use of at least one of MK-7, MK-8, MK-9 and MK-10, preferably in combination of at least one of a PUFA, either purified or in the form of fish oil or krill oil, for promoting at least one of cardiovascular health, bone health and cartilage health in humans and animals. A typical example of said use is for the preparation of a medicament for preventing or treating at least one of cardiovascular-, bone- and cartilage-related diseases or disorders. The medicament may be provided as a single medicament or as a kit.

In a third aspect, the present invention provides a pharmaceutical or nutraceutical product as defined hereinbefore
for use in a method of prophylaxis or
treatment of at least one of atherosclerosis, arteriosclerosis, osteoporosis, osteoarthritis or an inflammatory or degenerative disease of the cartilage.

Also described is counteracting certain negative aspects of fish oil, krill oil and PUFA containing foods and food supplements, thus reinforcing the beneficial aspects of said marine oils and PUFA for public health.

In a fourth aspect, the present invention provides a kit comprising the pharmaceutical or nutraceutical products comprising MK-7 and at least one of a PUFA, either purified or in the form of fish oil or krill oil as described above.

In a preferred embodiment of the invention, a PUFA, as used herein, is an omega-3 PUFA, for example eicosapentanoic acid (EPA) or docosahexaenoic acid (DHA).

These and other aspects of the present invention will be discussed in more detail in the following detailed description and examples.

### Brief description of the drawings

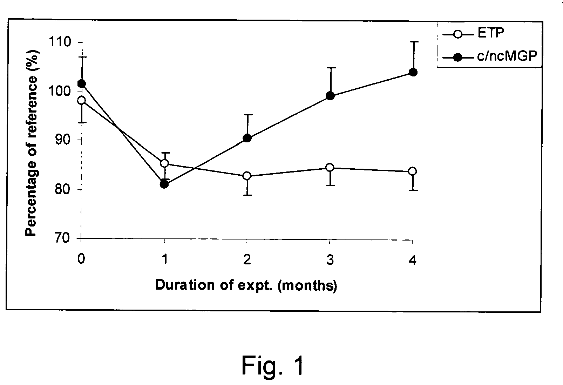

Figure 1 shows the endogenous thrombin potential (ETP), reflecting the total thrombin activity during coagulation. Carboxylated and non-carboxylated Matrix Gla-Protein (MGP) species were monitored using sandwich ELISAs based on conformation-specific antibodies with normal pooled plasma as a reference. The change in MGP carboxylation was significant after one month, but significance was lost during months 3 and 4. See Experiment 2 below.

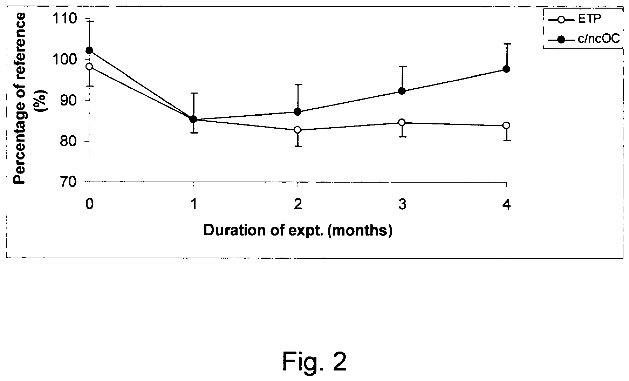

Figure 2 shows in the same experiment as in Figure 1 carboxylated and non-carboxylated osteocalcin species in serum using conformation-specific osteocalcin kits. The change in osteocalcin carboxylation was significant after one month, but significance was lost during months 3 and 4. See Experiment 3 below.

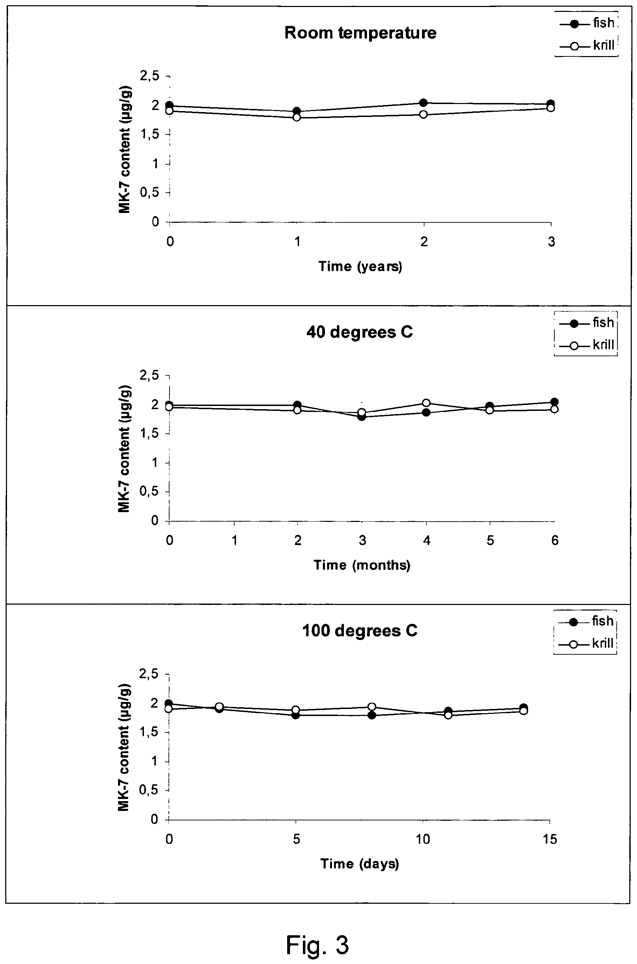

Figure 3 shows the exceptional stability of MK-7 dissolved in fish oil and krill oil. Samples were prepared in sealed glass bottles and kept in a dark place at room temperature, 40°C and 100°C, respectively. At regular times, samples were taken and analyzed for their MK-7 content. No loss of MK-7 was observed, even after 2 weeks at 100°C. See Experiment 4 below.

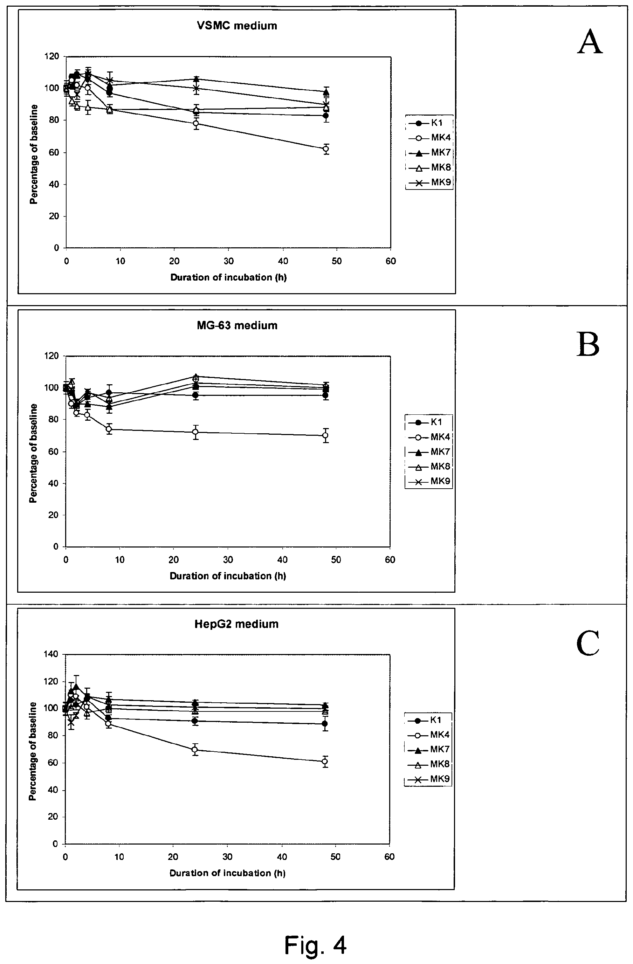

Figure 4 shows the stability of vitamins K₁, MK-4, MK-7, MK-8 and MK-9 in three cell culture media (used for growing the three different cell types described below, respectively) with no cells present. See Experiment 5 below.

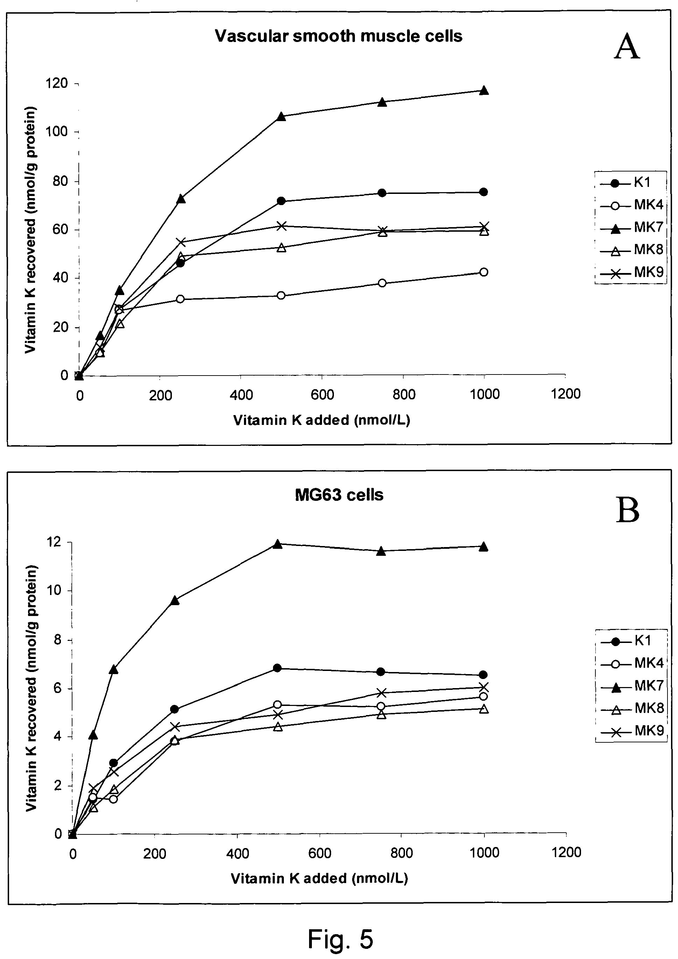

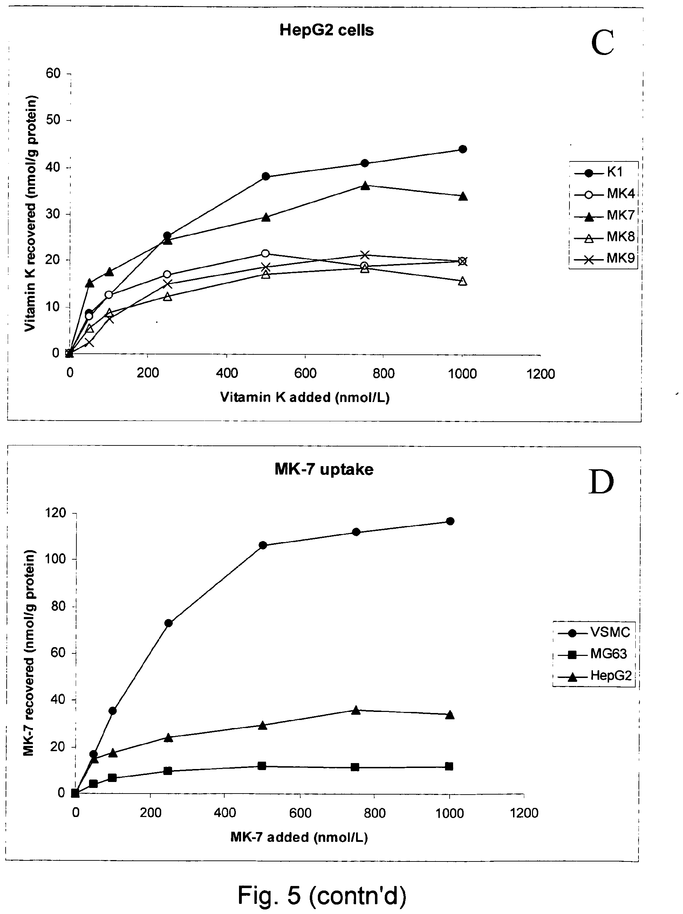

Figures 5A-C show dose-response curves for cellular vitamin K uptake in three different cell types; cells were grown until 80% confluence after which vitamin K (i.e., K₁, MK-4, MK-7, MK-8 and MK-9) was added in different concentrations. Figure 5D shows a plot of the MK-7 uptake in the three cell types at the same scale. See Experiment 6 below.

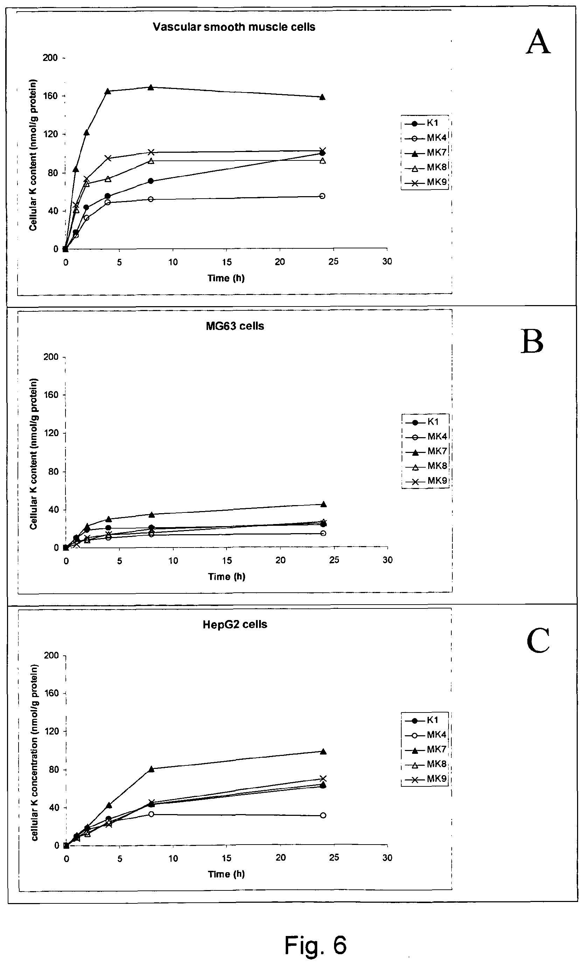

Figures 6A-C show the cellular uptake of vitamins K₁, MK-4, MK-7, MK-8 and MK-9 in the three different cell types as a function of time; cells were grown in the culture media decribed in Experiment 5. In all cases a mixture of these K vitamins was added to the cells after they had grown to 80% confluence. See Experiment 7 below.

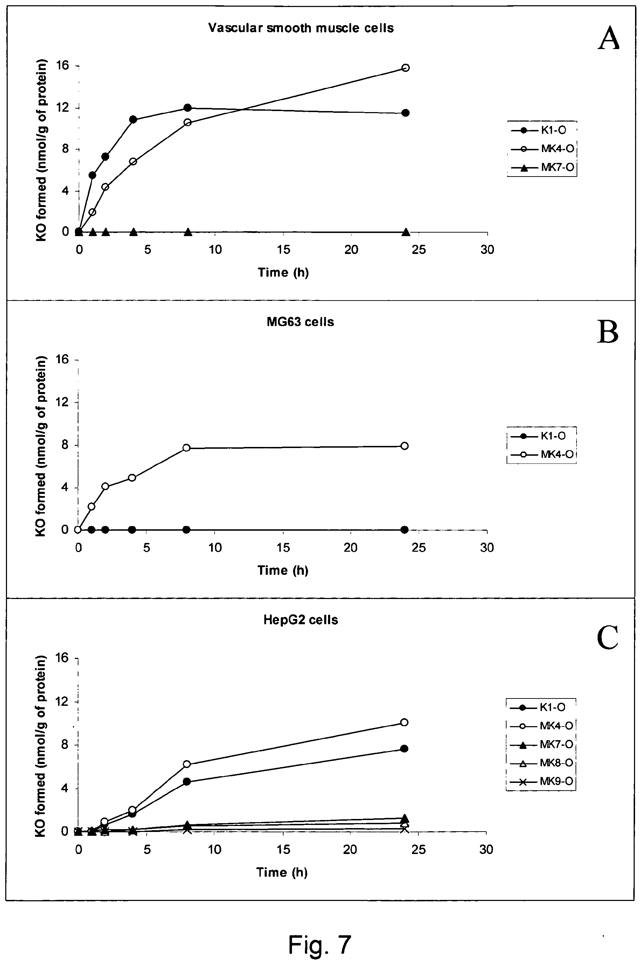

Figures 7A-C show the extent to which the effect of warfarin is bypassed by the various forms of vitamin K (as measured by K epoxide formation). The more KO formed the higher the extent of bypassing via the enzyme DT diaphorase. See Experiment 8.

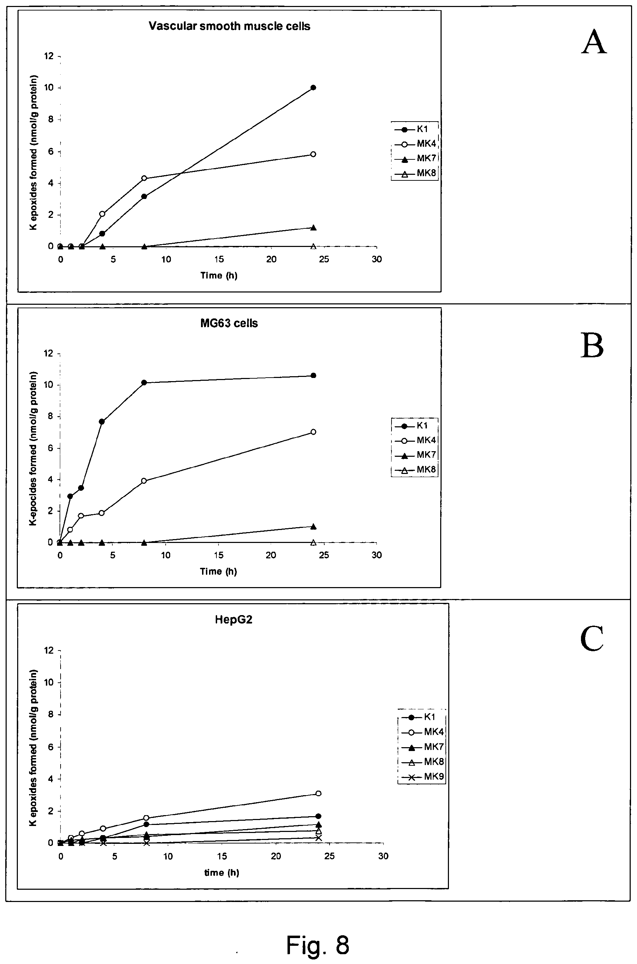

Figures 8A-C show the extent to which the various forms of vitamin K are utilized in the absence of warfarin, but under conditions of cell starvation. The more KO formed, the lesser a vitamin K species is recycled. See Experiment 9 below.

### Definitions

The term "polyunsaturated fatty acid" as used herein refers to any polyunsaturated fatty acid, whether in the form of a triglyceride, a physiologically acceptable ester, a free fatty acid or a physiologically acceptable salt of a polyunsaturated fatty acid. More specifically, the term is used to indicate omega-3 fatty acids, and omega-3 fatty acid-rich dietary oils such as fish oil and krill oil. In the literature, omega-3 fatty acids are also known as (n-3) polyunsaturated fatty acids or n-3 PUFA.

The term "krill oil" as used herein is meant to indicate all lipophilic components in krill and can be raw krill oil, purified krill oil, compounds from krill oil and derivatives of components from krill oil.

As used herein, fish oil and krill oil are collectively designated as "marine oils".

The term "vitamin K₂", as used herein, refers to naturally occurring menaquinones (MKs) or mixtures thereof.

The term "compound within the vitamin K₂ class of compounds", as used herein, refers to menaquinone-7 (MK-7).

The term "mainly MK-7" as used herein refers to compositions wherein at least 90% of all vitamin K₂ compounds in the composition, according to HPLC analysis, is MK-7.

### Detailed description of the invention

The present invention is primarily based on certain surprising findings in in-depth studies to the pharmacological properties of vitamin K₂, and in particular the higher menaquinones (MK-7, MK-8, MK-9 and MK-10), and combinations of these compounds with PUFAs, in pure form or in the form of fish oil or krill oil. These findings provide new possibilities and challenges for improved products in the pharmaceutical and nutraceutical field relating to the prophylaxis or treatment of certain cardiovascular-related, bone-related and cartilage-related disorders and, more in general, result in beneficial effects to the health of humans and animals.

These findings will be discussed in more detail below.

### Health effects of fish oil, krill oil and n-3 PUFA

Several health aspects of n-3 PUFA have been reported including a marked beneficial effect on cardiovascular disease. As has been reviewed by Van Schoonbeek et al. (Journal of Nutrition 133, 657-660 (2003)), the beneficial effects of n-3 PUFA and marine oils are based on multiple mechanisms including the reduction of blood platelet activity, a more favorable blood lipid profile and a reduction of blood coagulability.

The hypocoagulant effect of marine oil and n-3 PUFA is mainly attributed to a lowering of the hepatic vitamin K status and a concomitant decrease of the vitamin K-dependent clotting factors II, VII, IX, and X, leading to a decreased thrombin forming potential. C.M.A. Nieuwenhuys *et al.* concluded that *"Prolonged administration of n-3 but not n-6 PUFAs can lead to a hypocoagulable state of plasma through a reduced capacity of vitamin K-dependent thrombin generation with unchanged thrombin inactivation by antithrombin III".* A specific inhibition of vitamin K-dependent clotting factor synthesis was also reported by C. Leray et al. (Arteriosclerosis, Thrombosis, and Vascular Biology 22, 459-463 (2001)). This is consistent with the observation by L.J. Schurgers et al. (Journal of Lipid Research 43, 878-884 (2002), that some dietary oils may interfere with vitamin K absorption and metabolism in human volunteers. Direct evidence for interference with vitamin K metabolism was given by C.M.A. Nieuwenhuys et al. (Thrombosis Research 104, 137-147 (2001)) showing that fish oil and n-3 PUFAs decreased the vitamin K levels in the liver (i.e. the place where the clotting factors are synthesized). Furthermore, M. Andriamampandry et al. (Medical Sciences 321, 415-421 (1998)) demonstrated that the mild anticoagulant effect of fish oil was completely reversed by a slight increase of vitamin K₁ intake.

It has now surprisingly been found that vitamin K₂, notably MK-7, does not reverse the beneficial effect of fish oil. In dosages typically between 1 and 10 µg/day, MK-7 did not affect the extrinsic thrombin potential (ETP), which is the most sensitive measure for thrombosis risk. Hence, mixtures of vitamin K₂ with n-3 PUFA or marine oil combine the beneficial effects of both kinds of compounds and even worked synergistically in animal models and human volunteers. In doses of 100 µg MK-7 per day and higher the ETP lowering effect of marine oils was completely reversed. It should be noted that these doses of MK-7 relate to a marine oil intake of 5 grams per day. At higher fish or krill oil consumption also the dose required for interference with the ETP is higher.

### Cardiovascular health

Though a poor vitamin K status may have a mild anticoagulant (i.e. antithrombotic) effect, vitamin K is required in another aspect of vascular health. Matrix Gla-Protein (MGP) is a vitamin K-dependent protein synthesized in the arterial vessel wall. It is activated by vitamin K-dependent carboxylation and in its carboxylated form it acts as a powerful inhibitor of vascular calcification. Using conformation-specific antibodies, it was demonstrated by Schurgers et al. (Arteriosclerosis, Thrombosis and Vascular Biology 25, 1629-1633 (2005)) that atherosclerosis and vascular calcification are closely associated with poor vitamin K status of the vessel wall, and in a prospective clinical trial L.A.J.L.M. Braam et al. (Thrombosis & Haemostasis 91, 373-380 (2004)) found that high dose vitamin K-supplementation (1 mg/day) had major advantages for vascular elasticity. We have found that marine oil and n-3 PUFA not only favorably decrease the production of vitamin K-dependent proteins in the liver (the clotting factors) but also decrease the vitamin K-status of extrahepatic tissues and thus decrease the activation (by glutamate carboxylation) of extra-hepatic vitamin K-dependent proteins including osteocalcin and matrix Gla-protein (MGP). This is an unwanted side-effect of marine oils and n-3 PUFA with potential detrimental outcomes for cardiovascular health, for bone health and for the development of diseases of the cartilage. The diminished carboxylation of the vascular calcification inhibitor MGP, for instance, is a major risk factor for vascular calcification, hypertension, myocardial infarction and cardiovascular death.

It has now surprisingly been found that higher menaquinones, notably MK-7, in dosages between 1 and 10 µg/day do not counteract the mild anticoagulant effect of marine oils (as measured by the ETP), but at the same time they effectively stimulate MGP carboxylation to levels similar to or even above the level in subjects not using marine oil. This is described in Experiment 2 below. Hence, MK-7 is an essential ingredient to maximally benefit from marine oil and n-3 PUFA containing foods and food supplements directly applied for cardiovascular health.

### Bone health

Marine oils and n-3 PUFA have also been found to have beneficial effects on bone health. In experimental animal models such oils markedly decrease the loss of bone in ovariected mice and food-restricted rats (D. Sun et al. Journal of Bone and Mineral Research 18, 1206-1216 (2003); Sun et al. Bioscience, Biotechnology and Biochemistry 68, 2613-2615 (2004)). It has been reported by many authors that low vitamin K intake, poor vitamin K status and impaired osteocalcin carboxylation are risk factors for the development and progression of osteoporosis. See for instance: S.L. Booth et al. American Journal of Clinical Nutrition 77, 512-516 (2003), P. Szulc et al. Journal of Clinical Investigation 91, 1769-1774 (1993), C. Vermeer et al. European Journal of Nutrition 43, 325-325 (2004). It has also been demonstrated that increased intake of vitamin K results in a decreased rate of bone loss in postmenopausal women (L.A.J.L.M. Braam et al. Calcified Tissue International 73, 21-26 (2003), M. Shiraki et al. Journal of Bone and Mineral Research 15, 515-521 (2000)). The dosages used in these studies range between 1 mg/day for K₁ and 45 mg/day for K₂. In this light, the marine oil-induced decrease of osteocalcin carboxylation must be regarded as a detrimental side-effect which may in part obscure the benefits of marine oil for bone health. The reported high dose vitamin K treatment may counteract this side-effect, but will also annihilate the mild anticoagulant effect reported to be beneficial for cardiovascular disease prevention.

It has now surprisingly been found that the higher menaquinones, notably MK-7, in the dose rang between 1 and 10 µg/day did not counteract the mild anticoagulant effect of marine oils (as measured by the ETP), but at the same time effectively stimulated osteocalcin carboxylation to levels similar to or even above the level in subjects not using marine oil. This is described in Experiment 3 below. Hence MK-7 is an essential ingredient to maximally benefit from marine oil and n-3 PUFA containing foods and food supplements directly applied for bone health.

### Cartilage health

Marine oils and n-3 PUFA have also been found to counteract degenerative and inflammatory joint disease such as observed in osteoarthritis, rheumatoid arthritis, and ankylosing spondylitis. In cell culture studies it was demonstrated that supplementation with n-3 PUFA, but not n-6 PUFA, causes a decrease in both degenerative and inflammatory aspects of chondrocyte metabolism, whilst having no effect on normal tissue homeostasis (C.L. Curtis et al., Proc. Nutr. Soc. 61, 381-389 (2002)). Also population-based studies suggest a positive role of fish oil and n-3 PUFA for the treatment of osteoarthritis, rheumatoid arthritis and other diseases of the cartilage (see for instance: L.G. Cleland et al., Drugs 63, 845-853 (2003) and M.J. James et al., Prostaglandins, Leukotriens and Essential Fatty Acids 68, 399-405 (2003). As described above, marine oils and n-3 PUFA not only decrease the production of vitamin K-dependent proteins in the liver (the clotting factors) but also decrease the vitamin K-status of extrahepatic tissues and thus decrease the activation (by glutamate carboxylation) of extra-hepatic vitamin K-dependent proteins including osteocalcin and matrix Gla-protein (MGP). MGP is one of the most abundant proteins synthesized by cartilage, and vitamin K is needed for its activation by glutamate carboxylation. Poor MGP carboxylation has been associated with osteoarthritis, rheumatoid arthritis and ankylosing spondylitis. Clearly, a method is needed to counteract the marine oil-induced decrease of vitamin K status in cartilage.

It has now surprisingly been found that at nutritionally relevant doses MK-7 counteracts potentially negative aspects of marine oils on cartilage health. The fact that the higher menaquinones, notably MK-7, in the dose range between 1 and 10 µg/day do not counteract the mild anticoagulant effect of marine oil (as measured by the ETP), but at the same time effectively stimulate both osteocalcin carboxylation and MGP carboxylation to levels similar to or even above the level in subjects not using marine oil demonstrates that MK-7 is capable of providing optimal vitamin K status in bone, including the cartilage in the joints during marine oil supplementation. Hence MK-7 is an essential ingredient to maximally benefit from marine oils and n-3 PUFA containing foods and food supplements directly applied for cartilage health.

### Cell culture studies

In a series of cell culture experiments vitamin K uptake and metabolism under various conditions were investigated. The following forms of vitamin K were compared: K₁, MK-4, MK-7, MK-8 and MK-9. They were tested in three different cell types: the vascular smooth muscle cells, the osteoblast-like MG-63 cell line and the hepatocyte-like HepG2 cell line. In all experiments the vitamins were used as a mixture containing 1 µmol/L of each vitamin.

It was found that in all cells MK-7 is taken up better than other K vitamins. MK-8 and MK-9 are taken up somewhat less, K₁ and MK-4 much less.

It was also found that in the presence of the anticoagulant warfarin, the membrane-bound enzyme VKOR is blocked and thus vitamin K cannot be recycled. After utilization, it remains in the oxidized form (K-epoxide); the amounts of epoxides represent the extent of utilization. It was found that long-chain menaquinones and in particular MK-9 cannot be reduced, and thus not be used as a coenzyme for carboxylase under these conditions. The more water-soluble vitamins K₁ and MK-4, however, may use the cytoplasmic enzyme DT-diaphorase for reduction and interfere with oral anticoagulant treatment. Therefore, long-chain menaquinones are less likely to interfere with oral anticoagulant treatment than other forms of vitamin K. This is an important safety aspect, and consistent with animal experiments that have been published previously (Craciun, A.M., Groenen-van Dooren, M.M.C.L., Thijssen, H.H.W., Vermeer, C. (1998). Induction of prothrombin synthesis by K-vitamins compared in vitamin K-deficient and in brodifacoum-treated rats. Biochim. Biophys. Acta 1380, 75-81.

In the absence of warfarin and in rich medium, no epoxides are formed at all. If the concentration of fetal calf serum in the culture medium is decreased, the nutritional status of the cells becomes suboptimal. Also under these conditions MK-4 and K₁ epoxides were mainly found. The conclusion now is different than for the warfarin experiment, however: the long chain menaquinones are utilized preferentially and successfully compete with K₁ and MK-4 at the level of reduction by VKOR. Therefore, it can be concluded that long-chain menaquinones are preferentially used by for gamma-carboxylation of various proteins. This is consistent with previously published cell-free enzyme kinetic studies showing that the Michaelis constant (Km) for vitamin K decreases with increasing length of its side chain (Buitenhuis, H.C., Soute, B.A.M., Vermeer, C. (1990). Comparison of the vitamins K1, K2 and K3 as cofactors for the hepatic vitamin K-dependent carboxylase. Biochim. Biophys. Acta 1034, 170-175).

### Formulation of products and dosages

It has now been found that vitamin K₂ and polyunsaturated fatty acids can be formulated together in a stable formulation with long shelf life. For example, MK-7 was mixed with fish oil or krill oil in a wide range of concentrations and these compositions remained stable under these conditions for at least 2 weeks at 100°C, for 6 months at 40°C and for 3 years at room temperature. Details of these studies are shown in Experiment 4 below.

As defined above, the term "polyunsaturated fatty acid" (PUFA) refers to any PUFA in the form of a triglyceride, a physiologically acceptable ester, a free fatty acid or a physiologically acceptable salt thereof. Preferred compositions of PUFAs and menaquinones according to the present invention are PUFAs combined with mainly MK-7. More preferred composition of PUFA and MK substances according to the present invention are compositions wherein at least 95% of all vitamin K₂ compounds in the composition, according to HPLC analysis, is MK-7 with MK-6 as the main vitamin K₂ impurity. A typical ratio between MK-7 and MK-6 in compositions according to the present invention is in the range between 90 to 10 and 95 to 5.

Preferred compositions of a compound within the vitamin K₂ class of compounds and PUFA according to the present invention are compositions comprising a PUFA selected from n-3 PUFAs. More preferred n-3 PUFAs according to the present invention are eicosapentaenoic acid (EPA) which is a 20:5 acid and docosahexaenoic acid (DHA) which is a 22:6 acid.

An even more preferred n-3 PUFA component in products according to the present invention is a combination of EPA and DHA. The most preferred ratio of EPA : DHA is within the range of 2:1 to 1:2.

In the most preferred composition according to the present invention, EPA and DHA are in the form of triglycerides or ethyl esters.

The formulations of the products and kits of the invention are preferably administered systematically (e.g. orally or parenterally). The dosage form according to the present invention is an oral dosage form, i.e. capsules and tablets. The dosage and route of administration will depend on factors such as the age and sex of the individual, the severity and nature of the disorder or disease, and the like, and can easily be determined by a person skilled in the art, usually a physician, or the instructions leaflet of the manufacturer are to be followed which usually accompany the product.

Tablets and capsules can be prepared by any conventional manner known in the art. The capsules can for example be soft or hard gelatin capsules with in addition to vitamin K₂ or vitamin K₂ compounds and polyunsaturated fatty acid comprise of inactive pharmaceutically acceptable components, for example starch. Tablets according to the present invention may, for example, be prepared by direct compression or by compression of granules using conventional tablet machines. Tablets according to the present invention might in addition to vitamin K₂ or vitamin K₂ compounds and unsaturated fatty acid comprise of pharmaceutically acceptable inactive ingredients well known in the art. Such agents can for example be cellulose derivatives and magnesium stearate. Tablets according to the present invention can be coated with a gastric resistant coating, for example cellulose acetate phthalate.

The oral dosage forms, capsules and tablets, according to the present invention, have each a weight between 100 mg and 2 grams. The amount of vitamin K₂ compounds in each tablet or capsule may vary over a wide range, depending inter alia upon factors such as the severity and nature of the disease or disorder, and the condition, sex and age of the patient. The amount of a vitamin K₂ compound within the vitamin K₂ class of compounds in one tablet or capsule is between 1 and 10 µg, Dosages of MK-7 are between 1-10 µg per day.

The amount of unsaturated fatty acid in each tablet or capsule may also vary depending upon the nature of the unsaturated fatty acid, the severity and nature of the disease, age of the patient and frequency of administration of the tablet or capsule. The amount of unsaturated fatty acid in each tablet or capsule is typically between 300 and 1200 mg, preferably between 400 and 1000 mg, most preferably between 500 and 900 mg.

The data presented show that at dosages as high as 10 µg per day marine oil (at a dose of about 5 g) and MK-7 can be combined without interfering with the mild anticoagulant effect of marine oil. Thus, MK-7 dosages between about 1 and at least 10 µg (evidently, the higher the better within said range) have been proven to be beneficial when administered to a human or animal. Dosages as high as 100 µg per day are not applicable for the purpose described here, because these dosages were found to counteract the effect of warfarin, which is an even stronger inhibitor of blood clotting factor carboxylation than n-3 PUFAs are. See, Schurgers, L.J., Teunissen, K.J.F., Hamulyák, K., Knapen, M.H.J., Vik, H., Vermeer, C. Vitamin K-containing dietary supplements: comparison of synthetic vitamin K1 and natto-derived menaquinone-7. Blood 109 (2007) 3279-3283*.*

It will be understood that dosages of vitamin K and marine oils may also vary depending on the variety and quality of the marine oil. It is therefore recommended to use mixtures of marine oils in order to maintain a constant ratio in the compositions

It will also be understood that vitamin K₂ or a compound within the vitamin K₂ class of compounds can also be formulated separately and independently from one or more polyunsaturated fatty acids (including fish oil and krill oil), for example in a pharmaceutical composition or a nutraceutical composition (food supplement). Suitable pharmaceutical compositions and typical dosages are similar to those described above or are known to a person skilled in the art. Suitable nutraceutical compositions and typical dosages are described, e.g. in EP1153548.

The compositions according to the present invention can optionally comprise other pharmaceutically or nutraceutically active components; for example vitamins like vitamin D or vitamin D derivatives; active drug compounds such as bisphosphonates, typically alendronate; or cardiovascular drugs, such as ACE inhibitors, for example enalapril, angiotensin II receptor antagonists, for example losartan, beta-blockers, for example propranolol, plasma lipid reducing components, such as statins, typically simvastatin, and other drugs.

The use of MK-7 is generally the most preferred, as will be understood from the disclosure herein. Also described is MK-9, both because of its beneficial and promising properties and because of its availability since it is easily made synthetically.

The present invention will now be further described with reference to the following examples and experimental work.

### Example 1. Capsule comprising EPA ethyl ester, DHA ethyl ester and MK-7 (not within scope of invention)

An oil comprising 97% MK-7 and 3% MK-6 is mixed with EPA ethyl ester and DHA ethyl ester. Alpha-tocopherol is added, and the resulting oil is filled into hard gelatine 5 capsules.

Each capsule contains:

| | |
|---|---|
| MK-7 | 97 µg |
| MK-6 | 3 µg |
| EPA ethyl ester | 463 mg; |
| DHA ethyl ester | 375 mg |
| Alpha-tocopherol | 4 mg |

### Example 2. Cod liver oil comprising vitamin K₂

Vitamin K₂ is dissolved in cod liver oil (from EPAX, Norway) and filled into green glass bottles (250 ml) with a metal sealing. The bottles are labeled "Vitamin K₂ tran" for sale on the Norwegian market.

One daily dose (5ml) of this product contains:

| | |
|---|---|
| Vitamin A: | 250 µg |
| Vitamin D: | 10 µg |
| Vitamin K₂ (MK-7): | 10 µg |
| Fish oil: | 1.2 g, of which DHA was 0.4g and EPA was 0.6 g |
| Alpha-tocopherol: | 4 mg |

### Example 3: Capsule comprising krill oil and MK-7. (not within scope of invention)

Krill oil comprising polyunsaturated fatty acids in triglycerol form, phospholipids and other natural components (obtained from Enzymotec, Israel) is mixed with MK 7 and alpha-tocopherol. The resulting oil composition is filled into soft gel or hard gelatin capsules or prepared as an oil-containing granulate or solvent.

Each capsule contains:

| | |
|---|---|
| MK-7 | 97 µg |
| MK-6 | 3 µg |
| Krill oil | 700 mg |
| Alpha-tocopherol | 4 mg |

### Example 4: Krill oil comprising Vitamin K₂ (not within scope of invention)

Vitamin K₂ is dissolved in krill oil and filled into glass bottles (250 ml) with metal sealing. The bottles are labeled "Krilloljetran med Vitamin K" for sale on the Norwegian market or "Krill oil including vitamin K" for the US and UK market. One daily dose (5ml of krill oil) contains in the range between 5 and 500 µg Vitamin K₂.

### Example 5: Fish feed comprising krill oil and Vitamin K₂ (not within scope of invention)

Vitamin K₂ (100 mg) is dissolved in krill oil (1 kg). Fish feed (from Skretting AS, Stavanger, Norway) is ground. Ground fish feed (20 kg) is mixed with krill oil comprising Vitamin K₂ (1 kg) using a blender. Water was added , and the semi-wet material is sieved (3 mm) and tried for 24 hours at 40°C to obtain a fish feed granulate.

The feed granulate contains about 5% krill oil and 2-100 ppm of vitamin K₂.

### Example 6: MK-8 capsules (not within scope of invention)

An oil comprising MK-8 (HPCL analysis shows MK-8 higher than 70% of total Vitamin K2) is filled into soft gelatin capsules. Each capsule contains 5 µg of MK 8.

### Example 7: Capsules comprising EPA ethyl ester, DHA ethyl ester and blend of MK-8, MK-9 and MK-10. (not within scope of invention)

An oil comprising a blend of MK-8, MK-9, MK-10 is mixed with EPA ethyl ester and DHA ethyl ester. Alpha-tocopherol is added, and the resulting oil is filled into hard gelatin capsules.

Each capsule contains:

| | |
|---|---|
| MK-8, MK-9, MK-10 | 50 µg |
| EPA ethyl ester | 463 mg |
| DHA ethyl ester | 375 mg |
| Alpha-tocopherol | 4 mg |

### Example 8: Cod liver oil comprising MK8 (not within scope of invention)

MK8 (more than 65% according to HPLC analysis) is dissolved in cod liver oil and filled into green glass bottles (250 ml) with metal sealing.The bottles are labeled "Vitamin K2 tran" for sale on the Norwegian market.

One daily dose (5 ml) of this product contains:

| | |
|---|---|
| Vitamin A | 250 µg |
| Vitamin D | 10 µg |
| MK-8 | 10 µg |
| Omega-3 fatty acids | 1.2 g, of which DHA was 0.6 g and EPA was 0.4 g |
| dl-Alpha-tocopherylacetate | 6 mg |

### Experiment 1: The carboxylation degree of osteocalcin in osteoarthritis, osteopathy and rheumatoid arthritis.

Three patient groups were recruited and compared with age- and sex-matched healthy subjects. The vitamin K status of each subject was recorded by measuring the ratio between carboxylated and uncarboxylated osteocalcin (cOC/ucOC ratio), which is the most sensitive biomarker for extrahepatic vitamin K status (B. Panis et al., Bone 2006; 39:1123-1129). The patients suffering from osteoarthritis and chondropathy were relatively young and were compared with a young reference group (A), patients with rheumatoid arthritis were older and thus compared with an older reference group (B). Conformation-specific assays for osteocalcin were obtained from Takara (Japan). See Table 1 below.

This experiment demonstrates that the vitamin K status (expressed as the carboxylation degree of circulating osteocalcin) is significantly decreased in subjects with osteoarthritis, chondropathy and rheumatoid arthritis as compared to the healthy population.

**Table 1**

| | Control A | Control B | Osteoarthritis | Chondropathy | Rheumatoid Arthritis |
|---|---|---|---|---|---|
| Age (years) | 32 | 45 | 34 | 31 | 48 |
| Gender (% men) | 48 | 51 | 42 | 54 | 45 |
| Number per group | 30 | 30 | 27 | 49 | 29 |
| cOC/ucOC ratio | 1.39 | 1.28 | 1.04 | 0.71 | 0.38 |
| Range | 0.52-2.49 | 0.48-2.34 | 0.14-2.0 | 0.12-1.95 | 0.16-1.67 |
| Difference Cntr A | | | P < 0.05 | P < 0.005 | |
| Difference Cntr B | | | | | P < 0.002 |

### Experiment 2: MGP carboxylation during long term fish oil intake and the effects of MK-7 intake at 10 µg/day.

Twenty healthy males (43 ± 8 years of age) were enrolled in the study. The mean body mass index was 25.1 ± 3.5 kg/m². All subjects received 5 grams of fish oil (containing 35% EPA, 25% DHA and 10% other n-3 PUFA) per day during 4 months. During the last 3 months of the study all volunteers received additionally one soft gel capsule per day providing 10 µg of MK-7. Blood samples were collected in citrate every month to prepare platelet-rich plasma. The endogenous thrombin potential (ETP), reflecting the total thrombin activity during coagulation, was monitored according to H.C. Hemker et al. (Pathophysiology of Haemostasis and Thrombosis 2003;33:4-15). Carboxylated and non-carboxylated Matrix Gla-Protein (MGP) species were monitored using sandwich ELISAs based on conformation-specific antibodies obtained from VitaK BV (Maastricht, The Netherlands) with normal pooled plasma as a reference. See Figure 1. The ratio between carboxylated and non-carboxylated MGP is taken as a measure for vascular vitamin K status. All values are expressed as a percentage of those in the pooled reference plasma. The change in ETP was statistically significant (p<0.05) after one month and remained so during subsequent months. The change in MGP carboxylation was also significant after one month, but significance was lost during months 2, 3 and 4.

This experiment demonstrates that the fish-oil induced hypocoagulability is not counteracted by the low doses of MK-7 required to maintain MGP carboxylation at its original level.

### Experiment 3: Osteocalcin carboxylation during long term fish oil intake and the effects of MK-7 intake at 10 µg/day.

In the same experiment as described above, also serum was collected, in which carboxylated and non-carboxylated osteocalcin species were monitored using the conformation-specific osteocalcin kits from Takara (Japan). The change in osteocalcin carboxylation was significant after one month, but significance was lost during months 3 and 4. See Figure 2. This experiment demonstrates that the fish-oil induced hypocoagulability is not counteracted by the low doses of MK-7 required to maintain osteocalcin carboxylation at its original level.

### Experiment 4: Stability of MK-7 in fish oil and krill oil.

Compositions as described in Examples 2 and 4 (MK-7 dissolved in fish oil and krill oil, respectively) were prepared to a final concentration of 2 µg of MK-7 per g of oil. Samples were prepared in sealed glass bottles and kept in a dark place at room temperature, 40°C and 100°C, respectively. At regular times, samples were taken and analyzed for their MK-7 content. See Figure 3. No loss of MK-7 was observed, even after 2 weeks at 100°C. This experiment demonstrates the exceptional stability of MK-7 dissolved in fish oil and krill oil.

### Experiments 5-9: Cell culture studies

This series of cell culture experiments describe vitamin K uptake and metabolism under various conditions. The following forms of vitamin K were compared: K₁, MK-4, MK-7, MK-8 and MK-9. They were tested in three different cell types: the vascular smooth muscle cells, the osteoblast-like MG-63 cell line and the hepatocyte-like HepG2 cell line. In all experiments the vitamins were used as a mixture containing 1 µmol/L of each vitamin. All data are the means of triplicate experiments.

The composition (v/v) of the various culture media was:

| | HepG2 | VSMC | MG63 |
|---|---|---|---|
| EMEM (from Sigma) | 87% | 78% | 87% |
| Fetal Calf Serum | 10% | 20% | 10% |
| Penicillin/Streptamicin | 1% | 1% | 1% |
| L-glutamine | 1% | 1% | 1% |
| Sodium pyruvate | - | - | 1% |
| Non-essential amino acids | 1% | - | - |

### Experiment 5

Here we have checked the stability of the various forms of vitamin K (1 µM of each) in the three cell culture media with no cells present. The data are given in Figure 4. *Conclusion:* In all three growth media the vitamins K₁, MK-7, MK-8, and MK-9 were fairly stable over 48 hours, whereas MK-4 showed a decline to values between 70 and 80% of baseline for the first 24 hours and between 60 and 70% of baseline after 48 hours. The reason for this decline is still unclear, but conditions have to be chosen in such a way that the disappearance of MK-4 does not influence the data for cellular uptake of vitamin K. To keep MK-4 loss to a minimum, all further experiments were performed for not longer than 24 hours.

### Experiment 6

Cells were grown until 80% confluence after which vitamin K was added in different concentrations (see Figures 5A-C). The added vitamin K concentration is given as nmol/L of culture medium, the vitamin K recovered is given as nmol/g of cellular protein. Note the different scales for each vitamin.

### Conclusions:

1. The dose-response curves for all vitamins are horizontal between 500 and 1000 nmol/L; hence we work in saturating conditions and even the decline of MK4 (presently not understood) will not affect the outcomes of further studies.
2. Surprisingly, there was an enormous difference in vitamin K uptake by the various cells, with vascular smooth muscle cells giving the highest and MG63 the lowest values. This is demonstrated in Figure 5D, where we have plotted MK-7 uptake in the three cell types at the same scale.
3. In all systems, but notably in the extrahepatic cells, MK-7 was taken up surprisingly better than the other K vitamins. This is further demonstrated in the following experiments.

### Experiment 7

Cellular uptake of K vitamins was followed in time. In all cases a mixture of the various K vitamins (1 µmol/L final concentration in the culture medium) was added to the cells after they had grown to 80% confluence. The data are given in Figure 6. Surprisingly, MK-7 was taken up better than any of the other K vitamins. The mechanism behind this cellular preference for MK-7 is not quite clear.

### Conclusions:

1. Uptake levels off after 4 h and reaches plateau levels at 8 h;
2. The rate of uptake and the value of the plateau levels are different for each vitamin;
3. MK-7 is taken up better than any of the other vitamins in all three cell systems.

### Experiment 8

Under normal conditions, all forms of vitamin K are taken up in their quinone form, and they are reduced into the hydroquinone before being active as a cofactor for the enzyme gammaglutamylcarboxylase. During the carboxylation they are oxidized into epoxides, which can be reduced by the membrane-bound enzyme VKOR. VKOR forms the target for drugs known as coumarin derivatives, which inactivate the enzyme and thus block the recycling of K epoxides. Vitamin K quinones (and not the epoxides) can be reduced by a second enzyme system, known as DT diaphorase. Hence the action of coumarin derivatives, such as warfarin, is antagonized by extra vitamin K. Also, each molecule of vitamin K that is used in the presence of warfarin, is converted into the corresponding epoxide, which cannot be used any further. Hence the K epoxide concentration is a direct measure for the amount of vitamin K used during warfarin treatment. Coumarin derivatives are widely used as oral anticoagulants; their mode of action is based on the inhibition of clotting factor carboxylation in the liver. Interference with coumarin derivatives is thus a potential risk of vitamin K supplements. Therefore we have investigated whether and to which extend the various K vitamins interfere with warfarin. In the study described, 10 µmol/L of warfarin was added to the culture media 18 h before adding vitamin K. Also during incubation with K vitamins this concentration of warfarin was present. The results are shown in Figure 7.

In the MG63 cell line we could only identify MK-4 epoxide (MK4-O), in the other systems more K vitamins were detected in their epoxide form. In all cases it is obvious that the more water-soluble forms of vitamin K (MK-4 and K₁) are capable of bypassing the effect of warfarin, whereas at increasing hydrophobicity the interference with warfarin becomes less. The most important cell type in this series is the HepG2 cell line, since this represents the hepatocytes, the place where blood clotting factors are synthesized. It is obvious from Figure 7 that there is an inverse correlation between interference with warfarin and hydrophobicity of the vitamin K species. MK-9 epoxides were hardly formed. At this time MK-10 and MK-11 are not commercially available, but it may be expected by extrapolation that these long chain menaquinones will not interfere at all, and can be safely given as a supplement even to patients on oral anticoagulant (coumarin) treatment.

### Conclusions:

1. The short-chain K vitamins K₁ and MK-4 interfere substantially with warfarin, whereas interference by long-chain menaquinones is less.
2. Utilization of K-vitamins in the presence of warfarin is inversely related to the length of the side chain: MK-7 > MK-8 > MK-9.
3. On the basis of these experiments it may be expected that MK-9 and MK-10 will not interfere in a clinically relevant way with oral anticoagulant treatment.

### Experiment 9

After having demonstrated that long-chain menaquinones are absorbed well by the various cells, and that they are hardly used during warfarin treatment, it remains to be demonstrated that these vitamin K species are actively used under non-inhibited (warfarin-free) conditions. Since under standard cell culture conditions no epoxides are formed, we have applied sub-optimal growth conditions by decreasing the fetal calf serum in the culture medium to 1% (v/v). This resulted in substantial epoxide formation. The results are shown in Figure 8. Again, the short-chain vitamin K epoxides were found to accumulate, notably in the vascular smooth muscle MG63 cells. We interpret these data as preferential recycling of the long chain menaquinones under conditions of cell starvation. Alternatively, these data may be explained as no utilization of the long chain menaquinones. This was ruled out in an experiment in which VSMC and MG63 were grown at 1% FCS and with only one single form of vitamin K present (1 µmol/L). Using conformation-specific test kits for carboxylated osteocalcin (Takara, Japan) and carboxylated matrix Gla-protein (VitaK, the Netherlands) it was found that in vascular smooth muscle cells MGP carboxylation was stimulated by vitamin K₁. MK-4 and MK-7 to reach the following MGP levels in the culture medium after 24 hours incubation:
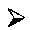 Carboxylated matrix Gla-protein in the presence of K1: 12.4 ng/mL, MK-4: 10.7 ng/mL, MK-7: 13.5 ng/mL.
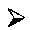 Carboxylated osteocalcin in the presence of K1: 3.2 ng/mL, MK-4: 4.1 ng/mL, MK-7: 3.9 ng/mL.

These data demonstrate that long chain menaquinones as well as K₁ and MK-4 are active cofactors for the vitamin K dependent carboxylase, and that the absence of epoxides must be the result of preferential recycling.

## Claims

1. A pharmaceutical or nutraceutical product for oral administration comprising menaquinone-7 (MK-7) in combination with at least one of a polyunsaturated fatty acid, either purified or in the form of a marine oil, wherein said product is a tablet formulation or a capsule formulation and wherein MK-7 is in the range of between 1-10 µg .

2. A pharmaceutical or nutraceutical product comprising MK-7 in combination with at least one of a polyunsaturated fatty acid, either purified or in the form of a marine oil, for use in a method for preventing or treating at least one of cardiovascular-, bone- and cartilage-related diseases or disorders in humans and animals, wherein the dosage of MK-7 is in the range of between 1-10 µg per day.

3. The product of claim 1 for use in a method for preventing or treating at least one of cardiovascular-, bone- and cartilage-related diseases or disorders.

4. The product of claim 1 for use in a method of prophylaxis or treatment of at least one of atherosclerosis, arteriosclerosis, osteoporosis, osteoarthritis or an inflammatory or degenerative disease of the cartilage.

5. The product of claim 1 which is provided in the form of a kit.

6. The product of claim 1, wherein said polyunsaturated fatty acid is in the form of fish oil or krill oil, or wherein said polyunsaturated fatty acid is a n-3 polyunsaturated fatty acid, preferably EPA or DHA or a mixture thereof, or wherein said fatty acid is a combination of EPA and DHA in the form of their triglycerides or ethyl esters.

7. The product for use according to claim 2, wherein said polyunsaturated fatty acid is in the form of fish oil or krill oil, or wherein said polyunsaturated fatty acid is a n-3 polyunsaturated fatty acid, preferably EPA or DHA or a mixture thereof, or wherein said fatty acid is a combination of EPA and DHA in the form of their triglycerides or ethyl esters.

8. The product according to claims 1 or 6, wherein said MK-7 and said fatty acid are provided in the same pharmaceutical composition.

## Patentansprüche

1. Pharmazeutisches oder nutrazeutisches Produkt zur oralen Verabreichung, umfassend Menachinon-7 (MK-7) in Kombination mit mindestens einer aus einer mehrfach ungesättigten Fettsäure, entweder gereinigt oder in Form eines marinen Öls, wobei das Produkt eine Tablettenformulierung oder eine Kapselformulierung ist und wobei MK-7 im Bereich von zwischen 1-10 µg liegt.

2. Pharmazeutisches oder nutrazeutisches Produkt, umfassend MK-7 in Kombination mit mindestens einer aus einer mehrfach ungesättigten Fettsäure, entweder gereinigt oder in Form eines marinen Öls, zur Verwendung in einem Verfahren zur Vorbeugung oder Behandlung mindestens einer aus kardiovaskulär-, knochen- und knorpelbezogenen Krankheiten oder Störungen bei Menschen und Tieren, wobei die Dosierung von MK-7 im Bereich von zwischen 1-10 µg pro Tag liegt.

3. Produkt nach Anspruch 1 zur Verwendung in einem Verfahren zur Vorbeugung oder Behandlung mindestens einer aus kardiovaskulär-, knochen- und knorpelbezogenen Krankheiten oder Störungen.

4. Produkt nach Anspruch 1 zur Verwendung in einem Verfahren zur Vorbeugung oder Behandlung von mindestens einer aus Atherosklerose, Arteriosklerose, Osteoporose, Arthrose oder einer entzündlichen oder degenerativen Erkrankung des Knorpels.

5. Produkt nach Anspruch 1, das in Form eines Kits bereitgestellt wird.

6. Produkt nach Anspruch 1, wobei die mehrfach ungesättigte Fettsäure in Form von Fischöl oder Krillöl vorliegt, oder wobei die mehrfach ungesättigte Fettsäure eine n-3 mehrfach ungesättigte Fettsäure, vorzugsweise EPA oder DHA oder eine Mischung davon ist, oder wobei die Fettsäure eine Kombination von EPA und DHA in Form ihrer Triglyceride oder Ethylester ist.

7. Produkt zur Verwendung nach Anspruch 2, wobei die mehrfach ungesättigte Fettsäure in Form von Fischöl oder Krillöl vorliegt, oder wobei die mehrfach ungesättigte Fettsäure eine n-3 mehrfach ungesättigte Fettsäure, vorzugsweise EPA oder DHA oder eine Mischung davon ist, oder wobei die Fettsäure eine Kombination von EPA und DHA in Form ihrer Triglyceride oder Ethylester ist.

8. Produkt nach den Ansprüchen 1 oder 6, wobei das MK-7 und die Fettsäure in derselben pharmazeutischen Zusammensetzung bereitgestellt werden.

## Revendications

1. Produit pharmaceutique ou nutriceutique pour administration orale comprenant de la ménaquinone-7 (MK-7) en association avec au moins l'un d'un acide gras polyinsaturé, soit purifié soit sous la forme d'une huile marine, dans lequel ledit produit est une formulation en comprimé ou une formulation en capsule et dans lequel la MK-7 est dans la plage entre 1 et 10 µg.

2. Produit pharmaceutique ou nutriceutique comprenant de la MK-7 en association avec au moins l'un d'un acide gras polyinsaturé, soit purifié soit sous la forme d'une huile marine, pour une utilisation dans un procédé de prévention ou de traitement d'au moins l'un de maladies ou de troubles cardiovasculaires, osseux et cartilagineux chez des humains et des animaux, dans lequel la posologie de la MK-7 est dans la plage entre 1 et 10 µg par jour.

3. Produit selon la revendication 1 pour une utilisation dans un procédé de prévention ou de traitement d'au moins l'un de maladies ou de troubles cardiovasculaires, osseux et cartilagineux.

4. Produit selon la revendication 1 pour une utilisation dans un procédé de prophylaxie ou de traitement d'au moins l'une de l'athérosclérose, de l'artériosclérose, de l'ostéoporose, de l'ostéoarthrite ou d'une maladie inflammatoire ou dégénérative du cartilage.

5. Produit selon la revendication 1 qui est fourni sous la forme d'un kit.

6. Produit selon la revendication 1, dans lequel ledit acide gras polyinsaturé est sous la forme d'huile de poisson ou d'huile de krill, ou dans lequel ledit acide gras polyinsaturé est un acide gras n-3 polyinsaturé, de préférence de l'EPA ou du DHA ou un mélange de ceux-ci, ou dans lequel ledit acide gras est une association d'EPA et de DHA sous la forme de leurs triglycérides ou esters éthyliques.

7. Produit pour une utilisation selon la revendication 2, dans lequel ledit acide gras polyinsaturé est sous la forme d'huile de poisson ou d'huile de krill, ou dans lequel ledit acide gras polyinsaturé est un acide gras n-3 polyinsaturé, de préférence de l'EPA ou du DHA ou un mélange de ceux-ci, ou dans lequel ledit acide gras est une association d'EPA et de DHA sous la forme de leurs triglycérides ou esters éthyliques.

8. Produit selon les revendications 1 ou 6, dans lequel ladite MK-7 et ledit acide gras sont fournis dans la même composition pharmaceutique.
